# EUROPEAN PATENT SPECIFICATION

(11) **EP 4 048 335 B1**
(45) Date of publication and mention of the grant of the patent: **12.08.2026**
(21) Application number: 20797562.4
(22) Date of filing: 15.10.2020
(51) Int. Cl.: A61M 1/00

(54) **FLUID BRIDGE CONFIGURED FOR USE VERTICALLY AND/OR UNDER COMPRESSION**
FLUIDBRÜCKE, KONFIGURIERT ZUR VERWENDUNG IN VERTIKALER RICHTUNG UND/ODER UNTER DRUCK
PONT DE FLUIDE CONÇU POUR ÊTRE UTILISÉ VERTICALEMENT ET/OU SOUS COMPRESSION

(30) Priority: 21.10.2019 US 201962924012 P
(43) Date of publication of application: 31.08.2022
(73) Proprietor: KCI Manufacturing Unlimited Company, Dublin, D01C4E0 (IE)
(72) Inventor: LONG, Justin Alexander, San Antonio, Texas 78265 (US); LOCKE, Christopher Brian, San Antonio, Texas 78265 (US)
(74) Representative: Simmons & Simmons
(86) International application number: PCT/IB2020/059723
(87) International publication number: WO 2021/079240

(56) References cited:
- WO-A1-2009/158130
- WO-A1-2018/212849
- WO-A1-2019/083607
- US-A1- 2018 153 570
- US-A1- 2019 231 602

## Description

### TECHNICAL FIELD

The invention set forth in the appended claims relates generally to tissue treatment systems and more particularly, but without limitation, to low-profile distribution components for providing negative-pressure therapy.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage". "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

Additionally, a firm-fitting wrap or elastic bandage may be used to apply compression to a limb or other tissue site in some instances. Such compression therapy may be particularly beneficial for treating venous disease, such as leg ulcers or oedema. Compression of a leg, for example, may increase the pressure within veins of the leg and, particularly the veins proximate to a surface of the leg. Veins proximate to a surface of a leg may also be known as superficial veins. Compression may generally encourage blood flow from superficial veins toward deeper veins where blood may be more readily carried out of a leg. An increase in pressure in veins can decrease swelling and can reduce symptoms of venous leg disease, for example, promoting healing.

While the clinical benefits of negative-pressure therapy and/or instillation therapy and/or compression therapy are widely known, improvements to therapy systems, components, and processes may benefit healthcare providers and patients.

WO2018/212849 disclose an absorbent negative-pressure dressing system for use with post-surgical breast wounds. The dressing comprises incision segments, such as a crown and an arm, and a bridge segment. The bridge segment forms a flow channel and comprises a manifold made of open cell foam and a dressing cover to provide fluid seal between the therapeutic environment and the local external environment.

US2018/0153570 discloses an apparatus for disrupting material at a tissue site in a negative pressure treatment system. The apparatus comprising a therapy system that can provide negative pressure therapy. The system comprises a dressing including a cover and a tissue interface. The tissue interface comprises a contact layer having a tissue-facing surface adapted to face the tissue site and an opposite surface adapted to face a retainer layer. The contact layer being an open cell, reticulated polyurethane foam such as GranuFoam^{®}.

WO2009/158130 discloses a negative pressure treatment system for providing a force to a desired area.

US2019/0231602 discloses a negative pressure treatment dressing comprising a perforated gel layer and perforated polymer film.

WO2019/083607 discloses an evaporative bridge dressing comprising a layer of wicking material.

### BRIEF SUMMARY

The claimed invention is defined by a fluid bridge according to claim 1. Preferred embodiments are claimed in the dependent claims 2-15.

### BRIEF DESCRIPTION OF THE DRAWINGS

Figure 1 is a functional block diagram of an example embodiment of a therapy system that can provide negative-pressure treatment in accordance with this specification;
Figure 2 is a schematic diagram of an example embodiment of the therapy system of Figure 1 configured to apply negative pressure to a tissue site utilizing an exemplary fluid bridge;
Figure 3A is a plan view of an exemplary fluid bridge;
Figure 3B is a schematic, cross-sectional view of the fluid bridge of Figure 3A taken along line 3B-3B;
Figure 3C is an exploded view of the fluid bridge of Figure 3A;
Figure 4 is a schematic, cross-sectional view of another exemplary fluid bridge;
Figure 5 is a chart of exemplary results illustrating performance of two vertically-oriented sample fluid bridges in comparison to a control sample; and
Figure 6 is a chart of additional exemplary results illustrating performance of two vertically-oriented sample bridges in comparison to a control sample when used under a compression wrap.

### DESCRIPTION OF EXAMPLE EMBODIMENTS

The following description of example embodiments provides information that enables a person skilled in the art to make and use the subject matter set forth in the appended claims, but it may omit certain details already well known in the art. The following detailed description is, therefore, to be taken as illustrative and not limiting. In the following description and claims, some values are expressed in non-SI units. For conversion to SI units, the following factors may be used:1 lb/ft3= 16.02 kg/m3; 1 inch =2.54 cm ; 1 Pounds per Square inch = 6.895 Pa.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

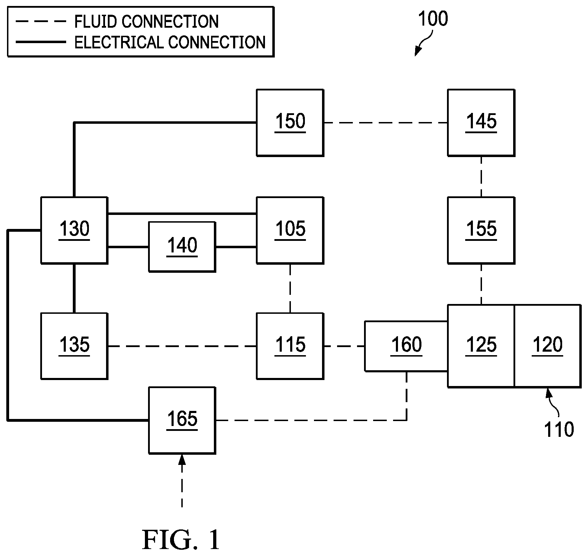

Figure 1 is a simplified functional block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. A tube, for example, is generally an elongated, flexible structure with a cylindrical lumen, but the geometry and rigidity may vary. Moreover, some fluid conductors may be molded into or otherwise integrally combined with other components. Distribution components may also include or comprise interfaces or fluid ports to facilitate coupling and decoupling other components. In some embodiments, for example, a dressing interface may facilitate coupling a fluid conductor to the dressing 110. For example, such a dressing interface may be a SENSAT.R.A.C.^{™} Pad, available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

In some embodiments, the therapy system 100 may also include a source of instillation solution. For example, a solution source 145 may be fluidly coupled to the dressing 110, as illustrated in the example embodiment of Figure 1. The solution source 145 may be fluidly coupled to a positive-pressure source, such as a positive-pressure source 150, a negative-pressure source, such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of Figure 1.

In some examples, a bridge 160 may fluidly couple the dressing 110 to the negative-pressure source 105, as illustrated in Figure 1. The therapy system 100 may also comprise a flow regulator, such as a regulator 165, fluidly coupled to a source of ambient air to provide a controlled or managed flow of ambient air. In some embodiments, the regulator 165 may be fluidly coupled to the tissue interface 120 through the bridge 160. In some embodiments, the regulator 165 may be positioned proximate to the container 115 and/or proximate a source of ambient air, where the regulator 165 is less likely to be blocked during usage.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mmHg (-667 Pa) and -500 mmHg (-66.7 kPa). Common therapeutic ranges are between -50 mmHg (-6.7 kPa) and -300 mmHg (-39.9 kPa). For example, the therapeutic range applied to the tissue site may be at least -75 mmHg in some embodiments.

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as fluid from a source of instillation solution, across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 120 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 120 may also vary according to needs of a prescribed therapy. For example, the tensile strength of foam may be increased for instillation of topical treatment solutions. The 25% compression load deflection of the tissue interface 120 may be at least 0.35 pounds per square inch, and the 65% compression load deflection may be at least 0.43 pounds per square inch. In some embodiments, the tensile strength of the tissue interface 120 may be at least 10 pounds per square inch. The tissue interface 120 may have a tear strength of at least 2.5 pounds per inch. In some embodiments, the tissue interface may be foam comprised of polyols, such as polyester or polyether, isocyanate, such as toluene diisocyanate, and polymerization modifiers, such as amines and tin compounds. In some examples, the tissue interface 120 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

The tissue interface 120 may be either hydrophobic or hydrophilic. In an example in which the tissue interface 120 may be hydrophilic, the tissue interface 120 may also wick fluid away from a tissue site, while continuing to distribute negative pressure to the tissue site. The wicking properties of the tissue interface 120 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 120 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 120 may further serve as a scaffold for new cell-growth, or a scaffold material may be used in conjunction with the tissue interface 120 to promote cell growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment. In some embodiments, the regulator 165 may control the flow of ambient air to purge fluids and exudates from the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and this descriptive convention should not be construed as a limiting convention.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

In some embodiments, the controller 130 may have a continuous pressure mode, in which the negative-pressure source 105 is operated to provide a constant target negative pressure for the duration of treatment or until manually deactivated. Additionally or alternatively, the controller may have an intermittent pressure mode. For example, the controller 130 can operate the negative-pressure source 105 to cycle between a target pressure and atmospheric pressure. In some examples, the target pressure may be set at a value of 135 mmHg for a specified period of time (e.g., 5 min), followed by a specified period of time (e.g., 2 min) of deactivation. The cycle can be repeated by activating the negative-pressure source 105, which can form a square wave pattern between the target pressure and atmospheric pressure.

In some example embodiments, the increase in negative pressure from ambient pressure to the target pressure may not be instantaneous. For example, the negative-pressure source 105 and the dressing 110 may have an initial rise time, which can vary depending on the type of dressing and therapy equipment being used. For example, the initial rise time for one therapy system may be in a range of about 20-30 mmHg/second and in a range of about 5-10 mmHg/second for another therapy system. If the therapy system 100 is operating in an intermittent mode, the repeating rise time may be a value substantially equal to the initial rise time.

In other examples, a target pressure can vary with time in a dynamic pressure mode. For example, the target pressure may vary in the form of a triangular waveform, varying between a negative pressure of 50 and 135 mmHg with a rise time set at a rate of +25 mmHg/min. and a descent time set at -25 mmHg/min. In other embodiments of the therapy system 100, the triangular waveform may vary between negative pressure of 25 and 135 mmHg with a rise time set at a rate of +30 mmHg/min and a descent time set at -30 mmHg/min.

In some embodiments, the controller 130 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 130, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

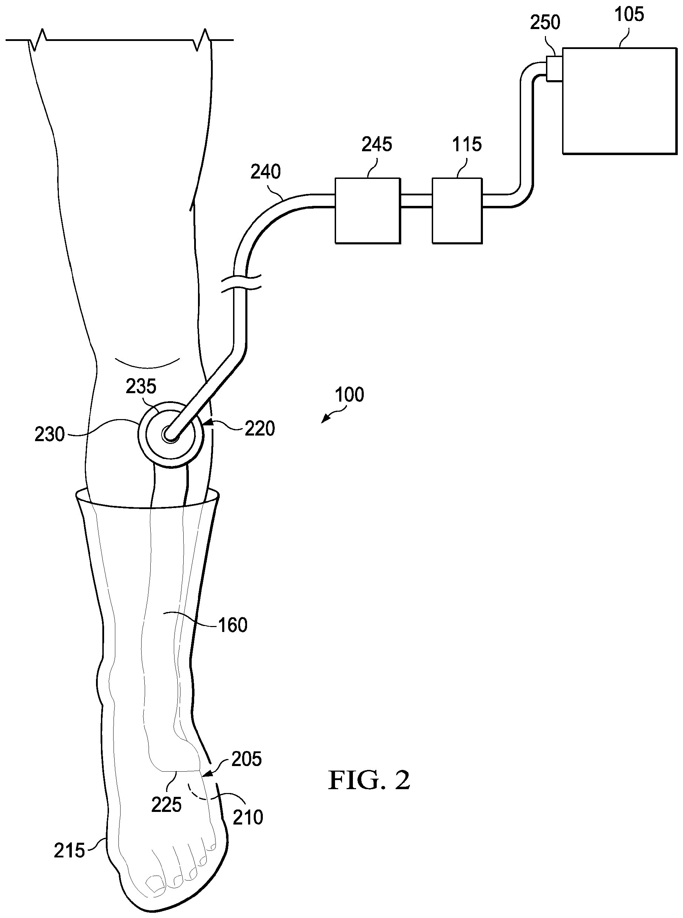

Figure 2 is a schematic diagram of an example embodiment of the therapy system of Figure 1 configured to apply negative pressure to a tissue site utilizing an exemplary fluid bridge 160, illustrating additional details that may be associated with some embodiments. The fluid bridge 160 may facilitate negative-pressure treatment of a tissue site 205, which in this illustration is on a patient's foot 210 and has limited access. In some embodiments, the treatment system 100 may be used with a tissue site having open access or limited access. An illustrative example of a limited-access tissue site may include a site under a compression garment 215.

The fluid bridge 160 may provide a low-profile path for negative pressure to be supplied to the tissue site 205 and thereby may increase patient comfort and enhance reliability of the negative-pressure supply to the tissue site 205. Because of the low profile of the fluid bridge 160, the fluid bridge 160 may readily be used with a compression garment 215. As such, the fluid bridge 160 may allow the patient the benefit of both negative-pressure treatment and compression treatment.

The fluid bridge 160 may have a first end 220 and a second end 225. The second end 225 may be placed proximate to the tissue site 205. In some embodiments, at least the second end 225 may be located under the compression garment 215. The first end 220 may typically be placed at a location on or near the patient that provides convenient access by the healthcare provider, such as a convenient location for applying negative pressure to the first end 220 of the fluid bridge 160. For example, first end 220 may be located away from the tissue site 205. In some embodiments, the first end 220 may be superior to the tissue site 205 (e.g. above the second end 225). In some embodiments, the first end 220 may be located external to the compression garment 215. As shown in Figure 2, the fluid bridge 160 may be oriented substantially longitudinally (e.g. when the patient is ambulatory). For example, the first end 220 may be located at a vertical height greater than that of the second end 225. In some embodiments, the first end 220 may have a negative-pressure-interface site 230 that is configured for receiving a negative-pressure interface 235, which may be a port, such as SENSAT.R.A.C.^{™} technology from Kinetic Concepts, Inc. of San Antonio, Texas. If the compression garment 215 is utilized, the fluid bridge 160 may typically extend from the tissue site (e.g. under the compression garment 215) to a place outside of the compression garment 215. For example, the first end 220 of the fluid bridge 160 typically may be located outside the compression garment and/or at a location without substantial compression. The actual length of the fluid bridge 160 may be varied to support use with a particular compression garment 215 or application.

Compression may be applied to the tissue site 205 and/or to portions of the fluid bridge 160 in some treatment applications. For example, the compression garment 215 may be placed over at least a portion of the bridge 160 and/or the tissue site 205 in some embodiments. For example, the compression garment 215 may cover and/or apply compression to at least the second end 225 of the fluid bridge 160. In some embodiments, the compression garment 215 may apply 20-60 mmHg compression, 40-60 mmHg compression, or about 40 mmHg compression. Exemplary compression garments may include a compression wrap or bandage or a compression boot.

In some embodiments, a negative-pressure delivery conduit 240 may fluidly couple the negative-pressure interface 235 to a negative-pressure source 105. The negative-pressure source 105 may be any device or means for supplying a reduced pressure, such as a vacuum pump or wall suction. While the amount and nature of negative pressure applied to a site will vary according to the application, the negative pressure may typically be between 5 mmHg and 500 mmHg or more typically between 25 mmHg to 200 mmHg. For vertical applications of the fluid bridge 160, such as is shown in Figure 2 on an ambulatory patient's leg, a specified minimum negative pressure may be necessary to ensure proper fluid flow. For example in some embodiments, a negative pressure of at least 125 mmHg may be a suitable minimum, but other pressures may be suitable for different situations. In some embodiments, the negative-pressure source 105 may provide about 125 mmHg negative pressure, or from 125 mmHg to 150 mmHg negative pressure may be provided. As used herein, "negative pressure" generally refers to a pressure less than the ambient pressure at a tissue site that is being subjected to treatment. In most cases, this negative pressure will be less than the atmospheric pressure at which the patient is located. Alternatively, the negative pressure may be less than a hydrostatic pressure at the tissue site. Unless otherwise indicated, values of pressure stated herein are gauge pressures. Although the terms "vacuum" and "negative pressure" may be used to describe the pressure applied to the tissue site, the actual pressure applied to the tissue site may be more than the pressure normally associated with a complete vacuum. Consistent with the use herein, an increase in negative pressure or vacuum pressure typically refers to a relative reduction in absolute pressure. In one illustrative embodiment, a V.A.C.^{®} Therapy Unit by Kinetic Concepts, Inc. of San Antonio may be used as the negative-pressure source 105.

The therapy system 100 typically may be configured to maintain therapeutic levels of negative pressure to the tissue site 205, for example at least 75 mmHg negative pressure at the tissue site 205. For example, the fluid bridge 160 may be configured so that the negative-pressure drop across the length of the fluid bridge 160 in vertical orientation may be no more than 45 mmHg and/or so that the negative-pressure drop across the length of the fluid bridge 160 may be no more than 50 mmHg when the fluid bridge 160 is vertically oriented and under compression. In some embodiments, although the head pressure (e.g. due to vertical height across fluid bridge 160) may still be present, the configuration of the fluid bridge 160 may ensure that there is substantially no additional negative-pressure drop due to compression. For example, the negative-pressure drop across the length of the fluid bridge 160 attributable to compression (e.g. the compression garment 215) may be 5 mmHg or less after the pressure head due to height of vertically oriented fluid bridge 160 has built (e.g. after about 30 hours of negative-pressure therapy).

Depending on the application, a plurality of devices may be fluidly coupled to the negative-pressure delivery conduit 240. For example, a fluid container 115 or a device 245 may be included in some embodiments. The device 245 may be representative of another fluid reservoir or canister to hold exudates and other fluids removed in some embodiments. Other representative examples of the device 245 that may be included on the negative-pressure delivery conduit 240 may include the following non-limiting examples: a pressure-feedback device, a volume detection system, a blood detection system, an infection detection system, a flow monitoring system, a temperature monitoring system, a filter, etc. In some embodiments, some of these devices may be formed integral to the negative-pressure source 105. For example, a negative-pressure port 250 on the negative-pressure source 105 may include a filter member that includes one or more filters, e.g., an odor filter.

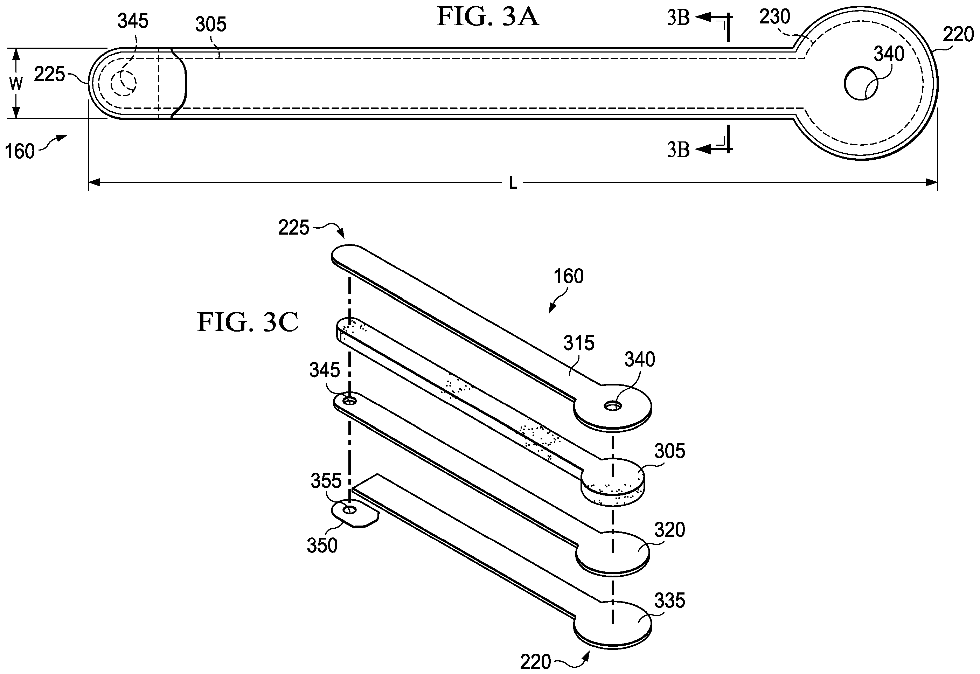

Figure 3A is a plan view of another example of the fluid bridge 160, illustrating additional details that may be associated with some embodiments. The fluid bridge 160 may be elongated, having a length L and a width W. In some embodiments, the length L of the fluid bridge 160 may be substantially greater than the width W. The comfort or function of the fluid bridge 160 may be enhanced by using a length L to width W ratio that involves having the length dimension greater than the width. For example, in some embodiments, the relationship may be L > 2W. In other illustrative embodiments, the relationship may be L > 6W. In other illustrative embodiments, the relationship may be L > 12W. In other illustrative embodiments, the relationship may be L > 15W. In some illustrative embodiments, L may be approximately 668 mm and W may be approximately 56 mm. In some embodiments, the length L of the fluid bridge 160 may be about 650-670 mm, for example 650 mm. In some embodiments, the width of the fluid bridge 160 may be 40-56 mm. In some embodiments, the width of the fluid bridge 160 may be about 40 mm.

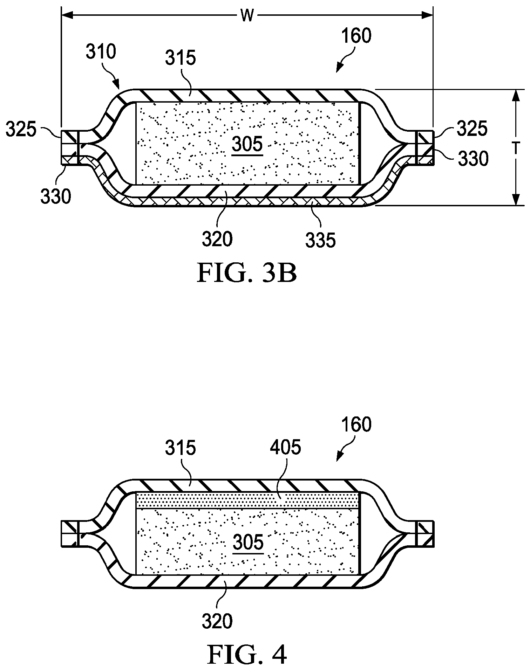

Figure 3B is a schematic, cross-sectional view of the fluid bridge 160 of Figure 3A taken along line 3B-3B, illustrating additional details that may be associated with some embodiments. As shown in Figure 3B, the fluid bridge 160 may also have a thickness T. In some embodiments, the fluid bridge 160 may preferably have a low profile (e.g., a small dimension T), which may be as small as possible while providing adequate fluid flow for negative-pressure therapy. For non-limiting examples, T may be 30 mm, 20 mm, 15 mm, 10 mm, 5 mm, or less. For example, T may be greater than 5 millimeters, 5-10 millimeters, 5-7 millimeters, or 6-7 millimeters.

In some embodiments, the fluid bridge 160 may comprise a support manifold 305 encompassed by an envelope 310. The envelope 310 may define an internal fluid flow path or pathway from the first end 220 to the second end 225 (as shown in Figure 3A). The envelope 310 may be made of a material that is impermeable to liquid and/or is substantially air-tight (e.g. allowing a vacuum to be drawn through the envelope). In some embodiments, the envelope 310 may comprise at least one vapor-transfer surface that is permeable to vapor. In some embodiments, the support manifold 305 may structurally support the envelope 310. For example, the support manifold 305 may be configured to maintain an open fluid flow path within the envelope 310 by maintaining space within the envelope 310 and/or preventing complete collapse of the envelope 310, for example due to negative pressure within the envelope 310 and/or application of compression forces on the fluid bridge 160. In some embodiments, the support manifold 305 may extend substantially the entire length L and/or width W and/or thickness T of the envelope 310, for example substantially filling the fluid flow path defined by the interior enclosed space of the envelope 310.

In some embodiments, the envelope 310 of Figure 3B may comprise a first encapsulating member 315 and a second encapsulating member 320, for example with a periphery of the first encapsulating member 315 coupled to the second encapsulating member 320 to form the envelope 310 with fluid pathway between the two ends of the fluid bridge 160. In Figure 3B, a periphery portion 325 of the first encapsulating member 315 and a periphery portion 330 of the second encapsulating member 320 may be coupled, such as by RF weld, to form the encapsulating envelope 310. The first encapsulating member 315 and the second encapsulating member 320 may be coupled using any technique, including without limitation welding (e.g., ultrasonic or RF welding), bonding, adhesives, cements, etc. The encapsulating envelope 310 may completely enclose the support manifold 305. Optionally, some embodiments may further comprise a moisture-removing device, such as wicking layer 335, which may be coupled to at least a portion of the encapsulating envelope 310 using any technique. For example, the wicking layer 335 may be coupled to an exterior side of the second encapsulating member 320 (e.g. opposite the support manifold 305).

Figure 3C is an exploded view of the fluid bridge 160 of Figure 3A, illustrating additional details associated with some embodiments. Figure 3C illustrates exemplary layers that may make up the fluid bridge 160. For example, in Figure 3C, the support manifold 305 may be located between the first encapsulating member 315 and the second encapsulating member 320. In some embodiments, the first encapsulating member 315 and the second encapsulating member 320 may each comprise a polymer film layer of liquid-impermeable material. The first encapsulating member 315 may be on a first side or surface of the fluid bridge 160, for example an outward-facing surface configured to face away from the patient. The first encapsulating member 315 may have a first aperture 340 formed proximate the first end 220. The first aperture 340 may allow fluid flow interaction between the internal flow path within the envelope 310 and an external negative-pressure source. Typically in use, the first aperture 340 may allow negative pressure application to the fluid bridge 160, for example with negative pressure entering the envelope 310 through the first aperture 340. The support manifold 305 may be disposed adjacent to the first encapsulating member 315. The second encapsulating member 320 may be disposed adjacent to the support manifold 305, opposite the first encapsulating member 315. For example, the second encapsulating member 320 may form the second, patient-facing side or surface of the fluid bridge 160. Thus, the support manifold 305 in Figure 3C may be located between and in contact with the first encapsulating member 315 and the second encapsulating member 320. The second encapsulating member 320 may be formed with a second aperture 345 proximate the second end 225. In some embodiments, the second aperture 345 may allow fluid interaction between the internal flow path within the envelope 310 and an external environment. Typically in use, the second aperture 345 may allow exudate from the tissue site to enter the fluid bridge 160 under negative pressure.

In some embodiments, a moisture-removing device may be disposed adjacent to the second encapsulating member 320, opposite the support manifold 305 (e.g. coupled to a patient-facing side of the second encapsulating member 320). As illustrated in the example of Figure 3C, the moisture-removing device may comprise a wicking layer 335 in some embodiments. In some embodiments, a releasable backing member or release liner 350 may be included on the second end 225 to releasably cover an adhesive. The releasable backing member 350 may be formed with an aperture 355 that aligns with the second aperture 345 in the second encapsulating member 320.

In some embodiments, the support manifold 305 may provide effective manifolding that distributes or collects fluid and/or negative pressure within the fluid bridge. For example, the support manifold 305 may receive negative pressure from a source at the first end 220 (e.g. at the first aperture 340) and distribute negative pressure through multiple apertures in the fluid bridge 160, which may have the effect of drawing fluid from the second end 225 towards the negative pressure source. The support manifold 305 may comprise any material capable of transferring negative pressure. In some embodiments, the support manifold 305 may be formed from the same material as the tissue interface 120. The support manifold 305 may have any thickness but typically may have a low profile, such as a thickness in the range of 3 - 20 millimeters, 5 - 10 millimeters, 5 - 7 millimeters, 6 - 7 millimeters, etc. In some embodiments, the support manifold 305 may be at least 5 millimeters thick. The thickness of the support manifold 305 may be varied to minimize or eliminate pressure points on the tissue site. The thickness of the support manifold 305 may also be selected to support fluid removal from the tissue site and transfer into a canister, such as the fluid canister 115 in Figure 2.

In some embodiments, the support manifold 305 may comprise an open-cell foam having a free volume in a range of about 18% to about 45%, a density of about 2.6-8.0 lb/ft³, about 80-250 pores per inch on average (e.g., as measured in the direction of compression), and/or average pore size of about 80-300 micron (e.g., as measured in the direction of compression), which may be particularly advantageous in a vertical orientation and/or under compression. For example, the denser foam may better resist the compressive effects when used under a compression garment and/or may better maintain fluid flow when under compression. In some embodiments, the density of the foam of the support manifold 305 may be about 3.9-4.8 lb/ft³. In some embodiments, the free volume of the foam may be in a range of about 18% to about 30%. In some embodiments, the free volume of the foam may be about 30%. In some embodiments, the average pore size of the support manifold 305 may be about 133-200 micron. In some embodiments, the support manifold 305 may have 120-150 pores per inch on average. For example, the support manifold 305 may comprise or consist essentially of a foam having 120-135 pores per inch on average. In some embodiments, the support manifold may have a 25% compression load deflection of at least 1.05 pounds per square inch and a 65% compression load deflection of at least 1.29 pounds per square inch. In some embodiments, the foam of the support manifold 305 may have a 25% compression load deflection of at least 1.75 pounds per square inch and a 65% compression load deflection of at least 2.15 pounds per square inch. In some embodiments, the foam of the support manifold 305 may have a 25% compression load deflection of about 1.05-1.75 pounds per square inch and a 65% compression load deflection of about 1.29-2.15 pounds per square inch.

In some embodiments, the support manifold 305 may be configured to maintain a thickness greater than 0.75 mm, 1mm, or 1.25 mm when vertically oriented, under compression wrap, and/or experiencing at least 125 mmHg negative pressure. In some embodiments, the support manifold 305 may be configured so that the negative-pressure drop across the length of the fluid bridge 160 due to compression (e.g. from a compression garment) may be 5 mmHg or less after the fluid bridge 160 has been in continuous use for 30 hours or more. In some embodiments, the support manifold 305 may be configured so that substantially any pressure drop may be due to the pressure head of the vertical column of exudate (e.g. without any substantial contribution from compression). In some embodiments, the fluid bridge 160 configuration may ensure that the width W of the fluid bridge 160 may decrease less than 1mm (e.g. about 0.8 mm) or less than 2 mm (e.g. about 1.9 mm) upon application of negative pressure (e.g. depending on the felting factor of the foam of the support manifold 305). In some embodiments, the fluid bridge 160 configuration may ensure that the width W of the fluid bridge 160 may decrease less than 10%, less than 5%, or about 2% or less upon application of negative pressure (e.g. 125 mmHg of negative pressure).

In some embodiments, the support manifold 305 may be formed by a felting process. Any porous foam suitable for felting may be used, including the example foams mentioned herein, such as found in GRANUFOAM^{™} dressing. In general, felting comprises a thermoforming process that permanently compresses a foam to increase the density of the foam while maintaining interconnected pathways. For example, in order to create felted foam, such as felted polyurethane, a foam blank of uncompressed material can be heated to an optimum forming temperature and then compressed. The felting process may alter certain properties of the original material, including pore shape and/or size, elasticity, density, and density distribution. For example, struts that define pores in the foam may be deformed during the felting process, resulting in flattened pore shapes. The deformed struts can also decrease the elasticity of the foam. The density of the foam is generally increased by felting. In some embodiments, contact with hot-press platens in the felting process can also result in a density gradient in which the density is greater at the surface and the pores size is smaller at the surface.

A felted foam may be characterized by a firmness factor, which is indicative of the compression of the foam. The firmness factor of a felted foam can be specified as the ratio of original thickness to final thickness. A compressed or felted foam may have a firmness factor greater than 1. The degree of compression may affect the physical properties of the felted foam. For example, felted foam has an increased effective density compared to a foam of the same material that is not felted. The felting process can also affect fluid-to-foam interactions. For example, as the density increases, compressibility or collapse may decrease. Therefore, foams which have different compressibility or collapse may have different firmness factors. In some example embodiments, a support manifold firmness factor can range from about 2 to about 5, preferably about 3 to about 5. For example, the firmness factor of the support manifold felted foam may be about 3 in some embodiments. There is a general linear relationship between firmness level, density, pore size (or pores per inch) and compressibility. For example, foam found in a GRANUFOAM^{™} dressing that is felted to a firmness factor of 3 will show a three-fold density increase and compress to about a third of its original thickness.

In some embodiments, a suitable foam blank (e.g. of pre-felted foam) for formation of the support manifold 305 may have 40-50 pores per inch on average, a density of 1.3-1.6 lb/ft³, a free volume of about 90% or more, an average pore size in a range of 400-600 micron, a 25% compression load deflection of at least 0.35 pounds per square inch, and/or a 65% compression load deflection of at least 0.43 pounds per square inch. In some embodiments, the foam blank may have a thickness greater than 10 mm, for example 10-35 mm, 10-25 mm, 10-20 mm, or 15-20 mm. In some embodiments, the foam blank may be felted to provide denser foam for the support manifold 305. For example, the foam blank may be felted to a firmness factor of 2-5. In some embodiments, the foam blank may be felted to a firmness factor of 3-5. Some embodiments may felt the foam blank to a firmness factor of 3.

Based for example on the foam selected for the fluid bridge 160 and/or the amount of felting, some fluid bridge 160 embodiments may be configured to reduce pressure drop across the length of the fluid bridge 160 (e.g. between the first end 220 and the second end 225 or the first aperture 340 and the second aperture 345) when the fluid bridge 160 is oriented substantially vertically and/or is under compression (e.g. due to application of a compression garment over at least a portion of the fluid bridge 160). For example, the negative-pressure drop across the length of the fluid bridge 160 (e.g. between the first end 220, where negative pressure is applied from the negative-pressure source 105, and the second end 225, where negative pressure is applied to the tissue site) may be no more than about 45 mmHg (e.g. if vertically oriented) or no more than about 50 mmHg (e.g. if vertically oriented and under compression). In some embodiments, the negative-pressure drop across the vertical length of the fluid bridge 160 may be less than 60 mmHg (e.g. when substantially vertically oriented) or less than 75 mmHg (e.g. when substantially vertically oriented and under compression). In some embodiments, the fluid bridge 160 may be configured to maintain at least 75 mmHg negative pressure at the second end 225 when 125 mmHg negative pressure is applied at the first end 220, even when used vertically and/or under compression.

The first encapsulating member 315 and the second encapsulating member 320 may be composed of any material that facilitates maintaining negative pressure within the encapsulating envelope formed from the first encapsulating member 315 and the second encapsulating member 320. In some embodiments, the first encapsulating member 315 and the second encapsulating member 320 may be formed of the same material as the cover 125. In some embodiments, the first encapsulating member 315 and the second encapsulating member 320 may comprise or consist essentially of a polyurethane film, but any suitable drape material may be readily used, such as any natural rubbers, polyisoprene, styrene butadiene rubber, chloroprene rubber, polybutadiene, nitrile rubber, butyl rubber, ethylene propylene rubber, ethylene propylene diene monomer, chlorosulfonated polyethylene, polysulfide rubber, polyurethane, EVA film, co-polyester, silicones, 3M Tegaderm^{®} drape material, or acrylic drape material, such as one available from Avery. In some embodiments, the polyurethane film may have a thickness of 10-100 micron. For example, the film may be 0.75 - 1.5 Mil or about 30 micron thick polyurethane. In some embodiments, at least one of the films may be hydrophilic. In some embodiments, the first encapsulating member 315 and the second encapsulating member 320 may not both comprise the same material and/or have the same thickness. Some embodiments may comprise a perforation (not shown) forming a calibrated flow into the envelope, for example located in proximity to the second end 225 of the fluid bridge 160. For example, the calibrated flow may be about 5 cc/min or less in some embodiments.

The wicking layer 335 or other moisture-removing device may be configured to pull moisture, e.g., perspiration, away from a patient's skin and may help to substantially reduce or prevent maceration of the patient's skin and enhance comfort. The extent of the moisture-removing device can be varied both laterally (width) and longitudinally (lengthwise). For example, the moisture-removing device may cover 100 percent or more than 90 percent, 80 percent, 70 percent, 60 percent, or 50 percent of the patient-facing second encapsulating member 315. The wicking layer 335 or moisture-removing device may be configured to pull moisture to a place where the moisture can evaporate more readily. In some embodiments, the wicking layer 335 may be a film, cloth-material drape, a non-woven fabric, a knitted polyester woven textile material, such as the one sold under the name InterDry^{®} AG material from Coloplast A/S of Denmark, GORTEX^{®} material, DuPont Softesse^{®} material, etc.

In some embodiments, apertures (not shown) may be formed on the second encapsulating member 320 that allow the negative pressure in the encapsulating envelope 310 to pull fluids from the wicking layer 335 into the support manifold 305. For example, apertures may be formed in the second encapsulating member 320 that allow the negative pressure within the encapsulating envelope 310 to pull fluids into the support manifold 305. In some embodiments, the apertures may comprise reduced-pressure valves configured to close in the absence of reduced pressure.

Figure 4 is a cross-section view of another example of a fluid bridge 160, illustrating additional details that may be associated with some embodiments. The fluid bridge 160 of Figure 4 may be similar to that of Figure 3B, but may also comprise a hydrophilic layer 405 located within the envelope. For example, the hydrophilic layer 405 may be oriented to face away from the patient when the fluid bridge 160 is applied to the tissue site, with the support manifold 305 between the hydrophilic layer 405 and the patient. In Figure 4, the hydrophilic layer 405 may be located between and in contact with the support manifold 305 and the first encapsulating member 315, while the support manifold 305 may be located between and in contact with the hydrophilic layer 405 and the second encapsulating member 320. The hydrophilic layer 405 in some embodiments may comprise hydrophilic open-cell foam configured to wick fluid away from the support manifold 305. In some embodiments, the outer surface of the envelope (e.g. the first encapsulating member 315) may comprise an evaporative material and/or be configured with a sufficiently thin thickness and/or a sufficiently vapor-permeable material to allow evaporation of some fluid within the envelope.

Some embodiments may be directed to a method of treating a tissue site with negative pressure. For example, methods of applying negative pressure to a tissue site may comprise the steps of: providing a fluid bridge having a support manifold with a first end and a second end; disposing the fluid bridge with the second end in proximity to the tissue site; providing a compression garment; disposing the compression garment to provide compression to the tissue site, wherein the second end is disposed under the compression garment; and applying negative pressure to the first end. In some embodiments, the second end may maintain at least -75 mmHg to the tissue site (e.g. 75 mmHg of negative-pressure). In some embodiments, the negative-pressure drop (e.g. change) between the first end and the second end may be no more than 50 mmHg. The step of disposing or placing the fluid bridge may comprise orienting the fluid bridge substantially vertically, in some embodiments. The step of disposing or placing the compression garment may comprise configuring the compression garment to apply about 20-60 mmHg or about 40-60 mmHg compression, in some embodiments. The step of applying negative pressure may in some embodiments comprise applying negative pressure of about 125 mmHg, at least 125 mmHg, or from about 125 to about 150 mmHg to the first end. In some embodiments, applying negative pressure may occur continuously for at least 60 hours (e.g. without negative-pressure drop of more than 50 mmHg and/or with at least 75 mmHg negative pressure being available at the second end). In some embodiments, the width of the fluid bridge may decease less than 1mm (e.g. about 0.8 mm) or less than 2 mm (e.g. about 1.8 mm) upon application of negative pressure (depending on the felting firmness factor of the support manifold). In some embodiments, the support manifold may comprise open-cell foam, for example as set forth in the example fluid bridge embodiments described above.

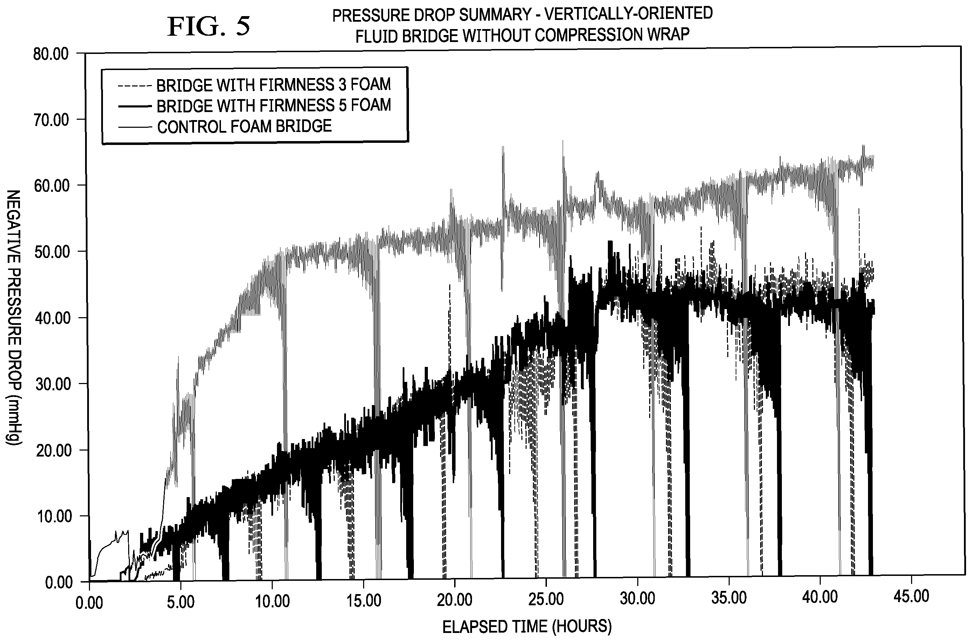

Figure 5 is a chart illustrating the effectiveness of exemplary fluid bridges having a felted foam versus a control bridge having un-felted foam in use for negative-pressure therapy. In Figure 5, the only difference between the three bridges tested was the firmness factor of the foam support manifold. One felted foam sample had a firmness factor of 3, and the other had a firmness factor of 5. The control bridge had a firmness factor of 1. All three bridges received 125 mmHg negative pressure. The fluid bridges in this example were all oriented vertically, and were not used under a compression wrap. As Figure 5 shows, both felted bridges produced significantly less negative-pressure drop across the length of the bridge (e.g. an improvement of about 10 mmHg or better). The negative-pressure drop for the control bridge eventually passed 50 mmHg and then 60 mmHg in the chart, which means that the therapeutic minimum goal of 75mmHg negative pressure was no longer being supplied to the tissue site. The felted bridges allowed more of the provided negative-pressure from the source to be applied to the tissue site, and ensured that at least 75 mmHg negative pressure was applied to the tissue site.

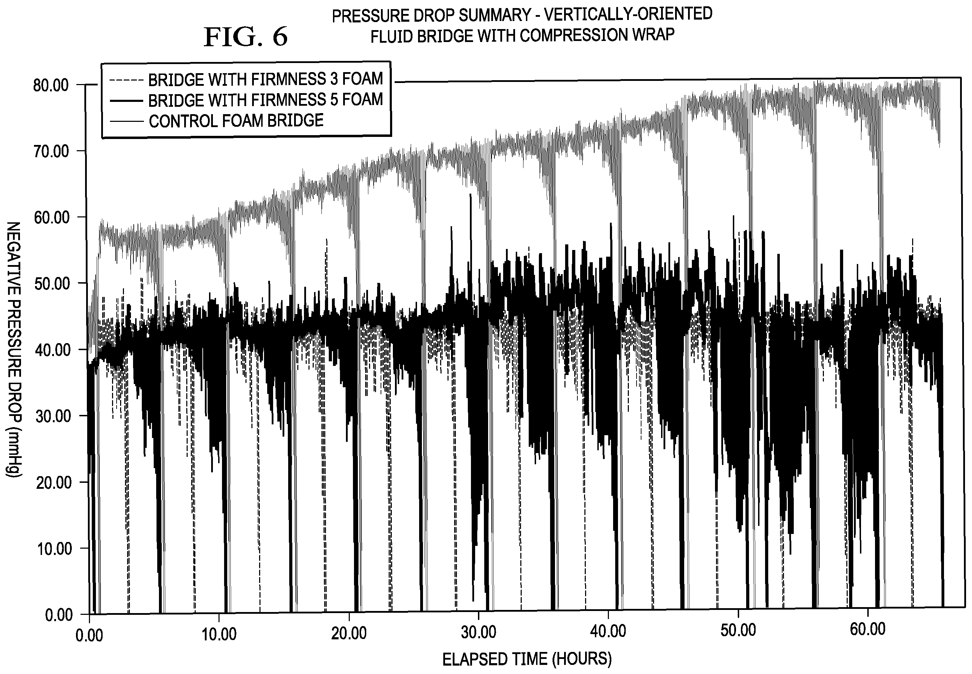

Figure 6 similarly illustrates in a chart the effectiveness of exemplary felted foam fluid bridges versus a control bridge with un-felted foam, when used for negative-pressure therapy. In Figure 6, the comparison occurs while the bridges are used with a compression wrap (e.g. with about 40 mmHg compression applied to the tissue site) and vertically oriented. Again, both felted bridges produced significantly less negative-pressure drop across the length of the bridge (e.g. an improvement of about 20 mmHg or better over the control bridge results). The negative-pressure drop for the un-felted bridge quickly passed 50 and 60 mmHg in the chart to approach 80 mmHg, which means that the therapeutic minimum goal of 75 mmHg negative pressure was no longer being supplied to the tissue site. The felted bridges allowed more of the provided negative-pressure from the source to be applied to the tissue site, and ensured that at least 75 mmHg negative pressure was applied to the tissue site. It may also be noted by comparing Figures 5 and 6 that, after the head pressure had time to build (e.g. about 30 hours), there was substantially no additional negative-pressure drop caused by the addition of compression. This may demonstrate the effectiveness of the felted foam fluid bridges when the negative-pressure therapy occurs in parallel to compressive therapy.

Some embodiments may be directed to a method of forming a fluid bridge, for example a fluid bridge configured for use vertically and/or under compression. For example, methods of forming a fluid bridge may comprise the steps of: selecting and/or providing an open-cell foam support manifold having a thickness of at least 5mm, a density of about 2.6-8.0 lb/ft3, a free volume of about 18-45% (e.g. 18-30%), and/or about 80-250 pores per inch on average; and encasing the support manifold in an envelope, wherein the support manifold supports the envelope to define an enclosed fluid pathway. In some embodiments, the average pore size of the open-cell foam of the support manifold may range from 80-300 micron. In some embodiments, the foam selected for the support manifold may be hydrophobic. The foam of some support manifold embodiments may comprise polyurethane foam. In some embodiments, the support manifold foam may have a thickness of about 5-7mm, may have a density of about 3.9-4.8 lb/ft3, may have average pore size of 133-200 micron, may on average have 120-150 pores per inch, may on average have 120-135 pores per inch, may have a 25% compression load deflection of at least 1.05 pounds per square inch and a 65% compression load deflection of at least 1.29 pounds per square inch, may have a 25% compression load deflection of at least 1.75 pounds per square inch and a 65% compression load deflection of at least 2.15 pounds per square inch, and/or may have a 25% compression load deflection of about 1.05-1.75 pounds per square inch and a 65% compression load deflection of about 1.29-2.15 pounds per square inch. In some embodiments, the foam of the support manifold may be selected to ensure that the fluid bridge maintains at least -75 mmHg at the second aperture when -125 mmHg is applied to the first aperture and/or that the negative-pressure drop across the length of the fluid bridge is no more than about 45 mmHg or about 50 mmHg (e.g. when the fluid bridge is oriented substantially vertically and/or is used under compression).

In some embodiments, the envelope may comprise a first surface and a second surface; the fluid bridge may comprise a first end and a second end; and the method may further comprise forming a first aperture located on the first surface in proximity to the first end and a second aperture located on the second surface in proximity to the second end. Some method embodiments may further comprise forming the open-cell foam support manifold, which may comprise: providing an open-cell foam blank (e.g. pre-felted foam) having thickness of at least 10 mm, 40-50 pores per inch on average, a free volume of about 90% or more, and/or density of 1.3-1.6 lb/ft3; and felting the foam blank. In some embodiments, felting the foam blank may comprise felting the foam blank to 2-5 firmness or to 3-5 firmness (for example, felted firmness factor 3). In some embodiments, the pre-felted foam (e.g. foam blank) may have average pore size of 400-600 micron; the pre-felted foam may have thickness of 10-35 mm; and/or the pre-felted foam may have 25% compression load deflection of at least 0.35 pounds per square inch, and 65% compression load deflection of at least 0.43 pounds per square inch.

Some method embodiments may further comprise attaching a moisture-removal layer adjacent to the second surface, opposite the support manifold. Some embodiments may further comprise forming a calibrated leak in the envelope of less than about 5cc/min located in proximity to the second end. Some method embodiments may further comprise: providing a hydrophilic foam layer; and disposing or placing the hydrophilic foam layer in proximity to the support manifold within the envelope. The first surface of the envelope may comprise an evaporative layer in some embodiments, and the hydrophilic layer may be disposed between the support manifold and the first surface.

The systems, apparatuses, and methods described herein may provide significant advantages. For example, some embodiments may provide a means for applying therapeutic negative pressure to a tissue site, and may be particularly effective if used in a vertical orientation and/or under compression. Accordingly, the patient may benefit from both compressive therapy and negative-pressure therapy in some applications. For example, simultaneous negative-pressure therapy and compressive therapy may be helpful in treating diabetic foot ulcer or venous leg ulcer. The configuration of the fluid bridge embodiments may allow for effective therapeutic negative-pressure therapy when the tissue site is located under a compression garment, resisting the compressive effects and maintaining sufficient open fluid flow pathways to maintain required negative pressure at the tissue site. The configuration of the fluid bridge embodiments may also allow for effective negative-pressure therapy at the tissue site despite the head pressure from vertical orientation. Pressure drop across the vertical length of the fluid bridge may be minimized, for example preventing severe pressure and fluid manifolding drops that could negatively impact negative-pressure therapy. In some embodiments, the pressure drop across the fluid bridge may be substantially similar (e.g. within about 5 mmHg) in a vertical orientation regardless of whether the fluid bridge is used under a compression garment or not. Some embodiments may substantially reduce or eliminate additional pressure drop induced by a compression garment. Some embodiments may minimize skin maceration issues.

While shown in a few illustrative embodiments, a person having ordinary skill in the art will recognize that the systems, apparatuses, and methods described herein are susceptible to various changes and modifications that fall within the scope of the appended claims. For example, the fluid bridge 160 may be used in other settings, for example without a compression garment and/or linking multiple tissue sites. In some embodiments, the fluid bridge 160 may be used for one or more tissue sites which may result in some or all of the patient's body weight compressing the fluid bridge.

Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 110, the container 115, or both may be separated from other components for manufacture or sale. In other example configurations, the controller 130 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art.

## Claims

1. A fluid bridge (160) for a system for treating a tissue site with negative pressure, comprising:
a first end (220), a second end (225), a support manifold (305) comprising open-cell foam having a density in a range of 41,7-128,2 kg/m³ and a free volume in a range of 18% to 45%, and an envelope (310) encompassing the support manifold (305), wherein the support manifold (305) supports the envelope (310) to maintain a fluid pathway within the envelope (310); and
whereby the first end (220) of the fluid bridge is adapted to fluidly couple the support manifold to
a negative-pressure source (105),
wherein the second end (225) is configured to be in proximity to and in fluid communication with the tissue site.

2. The fluid bridge (160) of claim 1, further comprising a compression garment (215) configured to cover at least the second end (225) of the fluid bridge (160).

3. The fluid bridge (160) of claim 2, wherein the compression garment (215) is configured to apply 20-60 mmHg compression.

4. The fluid bridge (160) of claims 2, wherein the compression garment (215) is configured to apply 40-60 mmHg compression.

5. The fluid bridge of claim 1, wherein:
the envelope (310) comprises a first surface and a second surface; and
the envelope (310) further comprises a first aperture (340) located on the first surface in proximity to the first end (220) and a second aperture (345) located on the second surface in proximity to the second end (225).

6. The fluid bridge of any of claims 1-3, wherein the open-cell foam has 31-98 pores per cm on average, 47-59 pores per cm on average, or 47-53 pores per cm on average.

7. The fluid bridge of any of claims 1-4 wherein the open-cell foam has an average pore size of 80-300 micron, or preferably 133-200 micron.

8. The fluid bridge of any of claims 1-5, wherein the open-cell foam of the support manifold (305) is hydrophobic.

9. The fluid bridge of any of claims 1-8, wherein the open-cell foam of the support manifold (305) has a density of 62.5-76.9 kg/m³.

10. The fluid bridge of any of claims 1-9, wherein the open-cell foam of the support manifold (305) has a 25% compression load deflection of at least 7.24 Pa and a 65% compression load deflection of at least 8.89 Pa, a 25% compression load deflection of at least 12.07 Pa and a 65% compression load deflection of at least 14.82 Pa, or a 25% compression load deflection in a range of 7.24-12.07 Pa and a 65% compression load deflection in a range of 8.89-14.82 Pa.

11. The fluid bridge of any of claims 1-10, wherein:
the envelope (310) comprises a first film layer (315) forming the first surface of the envelope (310) and a second film layer (320) forming the second surface of the envelope (310); and
the first film layer (315) and the second film layer (320) are coupled at a periphery to form the envelope (310).

12. The fluid bridge of any of claims 1-11, further comprising a wicking layer (335) adjacent to the second surface, opposite the support manifold (305).

13. The fluid bridge of any of claims 1-12, wherein the envelope (310) comprises a perforation providing controlled flow of less than 5cc/min located in proximity to the second end (225).

14. The fluid bridge of any of claims 1-13, further comprising a hydrophilic layer (405) adjacent to the support manifold (305) within the envelope (310).

15. The fluid bridge of any of claims 1-14, wherein the open-cell foam of the support manifold (305) comprises felted foam with a firmness factor of 2-5, or 3-5.

## Patentansprüche

1. Eine Fluidbrücke (160) für ein System zum Behandeln einer Gewebestelle mit Unterdruck, aufweisend:
ein erstes Ende (220), ein zweites Ende (225), einen Stützverteiler (305), aufweisend offenzelligen Schaum, der eine Dichte im Bereich von 41,7-128,2 kg/m³ und ein freies Volumen im Bereich von 18 % bis 45 % hat, und eine Hülle (310), die den Stützverteiler (305) umschließt, wobei der Stützverteiler (305) die Hülle (310) stützt, um einen Fluidweg innerhalb der Hülle (310) aufrechtzuerhalten; und
wobei das erste Ende (220) der Fluidbrücke angepasst ist, um den Stützverteiler mit einer Unterdruckquelle (105) fluidisch zu koppeln,
wobei das zweite Ende (225) konfiguriert ist, um in einer Nähe der Gewebestelle und in Fluidverbindung mit dieser zu sein.

2. Die Fluidbrücke (160) nach Anspruch 1, ferner aufweisend ein Kompressionskleidungsstück (215), das konfiguriert ist, um mindestens das zweite Ende (225) der Fluidbrücke (160) zu bedecken.

3. Die Fluidbrücke (160) nach Anspruch 2, wobei das Kompressionskleidungsstück (215) konfiguriert ist, um eine Kompression von 20-60 mmHg anzuwenden.

4. Die Fluidbrücke (160) nach Anspruch 2, wobei das Kompressionskleidungsstück (215) konfiguriert ist, um eine Kompression von 40-60 mmHg anzuwenden.

5. Die Fluidbrücke nach Anspruch 1, wobei:
die Hülle (310) eine erste Oberfläche und eine zweite Oberfläche aufweist; und
die Hülle (310) ferner eine erste Öffnung (340), die sich auf der ersten Oberfläche in der Nähe des ersten Endes (220) befindet, und eine zweite Öffnung (345), die auf der zweiten Oberfläche in der Nähe des zweiten Endes (225) angeordnet ist, aufweist.

6. Die Fluidbrücke nach einem der Ansprüche 1 bis 3, wobei der offenzellige Schaum im Durchschnitt 31-98 Poren pro cm, im Durchschnitt 47-59 Poren pro cm oder im Durchschnitt 47-53 Poren pro cm hat.

7. Die Fluidbrücke nach einem der Ansprüche 1 bis 4, wobei der offenzellige Schaum eine durchschnittliche Porengröße von 80-300 Mikron oder vorzugsweise 133-200 Mikron hat.

8. Die Fluidbrücke nach einem der Ansprüche 1 bis 5, wobei der offenzellige Schaum des Stützverteilers (305) hydrophob ist.

9. Die Fluidbrücke nach einem der Ansprüche 1 bis 8, wobei der offenzellige Schaum des Stützverteilers (305) eine Dichte von 62,5-76,9 kg/m³ hat.

10. Die Fluidbrücke nach einem der Ansprüche 1 bis 9, wobei der offenzellige Schaum des Stützverteilers (305) eine 25 %ige Stauchhärte von mindestens 7,24 Pa und eine 65 %ige Stauchhärte von mindestens 8,89 Pa, eine 25 %ige Stauchhärte von mindestens 12,07 Pa und eine 65 %ige Stauchhärte von mindestens 14,82 Pa, oder eine 25 %ige Stauchhärte im Bereich von 7,24-12,07 Pa und eine 65 %ige Stauchhärte im Bereich von 8,89-14,82 Pa hat.

11. Die Fluidbrücke nach einem der Ansprüche 1 bis 10, wobei:
die Hülle (310) eine erste Folienschicht (315), die die erste Oberfläche der Hülle (310) bildet, und eine zweite Folienschicht (320), die die zweite Oberfläche der Hülle (310) bildet, aufweist; und
die erste Folienschicht (315) und die zweite Folienschicht (320) an einem Umfang gekoppelt sind, um die Hülle (310) zu bilden.

12. Die Fluidbrücke nach einem der Ansprüche 1 bis 11, ferner aufweisend eine Dochtwirkungsschicht (335) angrenzend an die zweite Oberfläche, gegenüberliegend des Stützverteilers (305).

13. Die Fluidbrücke nach einem der Ansprüche 1 bis 12, wobei die Hülle (310) eine Perforation, die einen kontrollierten Durchfluss von weniger als 5 cc/min bereitstellt, die in der Nähe des zweiten Endes (225) angeordnet ist, aufweist.

14. Die Fluidbrücke nach einem der Ansprüche 1 bis 13, ferner aufweisend eine hydrophile Schicht (405) angrenzend an den Stützverteiler (305) innerhalb der Hülle (310).

15. Die Fluidbrücke nach einem der Ansprüche 1 bis 14, wobei der offenzellige Schaum des Stützverteilers (305) Filzschaum mit einem Festigkeitsfaktor von 2-5 oder 3-5 aufweist.

## Revendications

1. Pont à fluide (160) pour un système destiné à traiter un site tissulaire par pression négative, comprenant :
une première extrémité (220), une seconde extrémité (225), un collecteur de support (305) comprenant une mousse à cellules ouvertes ayant une densité comprise entre 41,7 et 128,2 kg/m³ et un volume libre compris entre 18 % et 45 %, et une enveloppe (310) entourant le collecteur de support (305), dans lequel le collecteur de support (305) supporte l'enveloppe (310) pour maintenir un chemin de passage de fluide à l'intérieur de l'enveloppe (310) ; et
moyennant quoi la première extrémité (220) du pont à fluide est adaptée pour accoupler fluidiquement le collecteur de support à une source de pression négative (105),
dans lequel la seconde extrémité (225) est conçue pour être à proximité du site tissulaire et en communication fluidique avec celui-ci.

2. Pont à fluide (160) selon la revendication 1, comprenant en outre un vêtement de compression (215) conçu pour recouvrir au moins la seconde extrémité (225) du pont à fluide (160).

3. Pont à fluide (160) selon la revendication 2, dans lequel le vêtement de compression (215) est conçu pour appliquer une compression de 20 à 60 mmHg.

4. Pont à fluide (160) selon la revendication 2, dans lequel le vêtement de compression (215) est conçu pour appliquer une compression de 40 à 60 mmHg.

5. Pont à fluide selon la revendication 1, dans lequel :
l'enveloppe (310) comprend une première surface et une seconde surface ; et
l'enveloppe (310) comprend en outre une première ouverture (340) située sur la première surface à proximité de la première extrémité (220) et une seconde ouverture (345) située sur la seconde surface à proximité de la seconde extrémité (225).

6. Pont à fluide selon l'une quelconque des revendications 1 à 3, dans lequel la mousse à cellules ouvertes possède 31 à 98 pores par cm en moyenne, 47 à 59 pores par cm en moyenne, ou 47 à 53 pores par cm en moyenne.

7. Pont à fluide selon l'une quelconque des revendications 1 à 4, dans lequel la mousse à cellules ouvertes possède une taille moyenne de pore de 80 à 300 microns, ou de préférence de 133 à 200 microns.

8. Pont à fluide selon l'une quelconque des revendications 1 à 5, dans lequel la mousse à cellules ouvertes du collecteur de support (305) est hydrophobe.

9. Pont à fluide selon l'une quelconque des revendications 1 à 8, dans lequel la mousse à cellules ouvertes du collecteur de support (305) possède une densité de 62,5 à 76,9 kg/m³.

10. Pont à fluide selon l'une quelconque des revendications 1 à 9, dans lequel la mousse à cellules ouvertes du collecteur de support (305) possède une déflexion sous charge de compression à 25 % d'au moins 7,24 Pa et une déflexion sous charge de compression à 65 % d'au moins 8,89 Pa, une déflexion sous charge de compression à 25 % d'au moins 12,07 Pa et une déflexion de charge de compression à 65 % d'au moins 14,82 Pa, ou une déflexion sous charge de compression à 25 % dans une plage allant de 7,24 à 12,07 Pa et une déflexion sous charge de compression à 65 % comprise dans une plage allant de 8,89 à 14,82 Pa.

11. Pont à fluide selon l'une quelconque des revendications 1 à 10, dans lequel :
l'enveloppe (310) comprend une première couche de film (315) formant la première surface de l'enveloppe (310) et une seconde couche de film (320) formant la seconde surface de l'enveloppe (310) ; et
la première couche de film (315) et la seconde couche de film (320) sont accouplées au niveau d'une périphérie pour former l'enveloppe (310).

12. Pont à fluide selon l'une quelconque des revendications 1 à 11, comprenant en outre une couche à effet mèche (335) adjacente à la seconde surface, opposée au collecteur de support (305).

13. Pont à fluide selon l'une quelconque des revendications 1 à 12, dans lequel l'enveloppe (310) comprend une perforation fournissant un écoulement contrôlé inférieur à 5 cm³/min située à proximité de la seconde extrémité (225).

14. Pont à fluide selon l'une quelconque des revendications 1 à 13, comprenant en outre une couche hydrophile (405) adjacente au collecteur de support (305) à l'intérieur de l'enveloppe (310).

15. Pont à fluide selon l'une quelconque des revendications 1 à 14, dans lequel la mousse à cellules ouvertes du collecteur de support (305) comprend une mousse feutrée avec un facteur de fermeté de 2 à 5 ou 3 à 5.
